# EUROPÄISCHE PATENTSCHRIFT

(11) **EP 4 215 153 B1**
(45) Veröffentlichungstag und Bekanntmachung des Hinweises auf die Patenterteilung: **30.04.2025**
(21) Anmeldenummer: 22215962.6
(22) Anmeldetag: 22.12.2022
(51) Int. Cl.: A61C 17/20

(54) **ULTRASCHALL-BEHANDLUNGSGERÄT UND ZUGEHÖRIGER ZUNGENBEHANDLUNGSAUFSATZ**
ULTRASONIC TREATMENT APPARATUS AND RELATED TONGUE TREATMENT ATTACHMENT
APPAREIL DE TRAITEMENT PAR ULTRASONS ET ACCESSOIRE DE TRAITEMENT DE LA LANGUE ASSOCIÉ

(30) Priorität: 21.01.2022 DE 102022101416
(43) Veröffentlichungstag der Anmeldung: 26.07.2023
(73) Patentinhaber: emmi EMAG AG, 64546 Mörfelden-Walldorf (DE)
(72) Erfinder: Emekci, Cüneyt, 61118 Bad Vilbel (DE); Emekci, Can, 64546 Mörfelden-Walldorf (DE)
(74) Vertreter: Glück Kritzenberger Patentanwälte PartGmbB

(56) Entgegenhaltungen:
- CN-A- 110 314 006
- DE-A1- 102019 000 770

## Beschreibung

Die Erfindung betrifft ein Ultraschall-Behandlungsgerät gemäß Patentanspruch 1 und einen zugehörigen Zungenbehandlungsaufsatz gemäß Patentanspruch 10.

Ultraschall-Behandlungsgeräte für die Mund- und/oder Zahnhygiene, insbesondere Ultraschall-Zahnbürsten sind bereits bekannt.

Insbesondere sind bereits Ultraschall-Zahnbürsten bekannt, die ein Handstück bzw. ein Griffteil aufweisen, an dem ein Behandlungsaufsatz, insbesondere ein Ultraschall-Zahnbürstenaufsatz angeordnet ist. Dieser Aufsatz ist vorzugsweise abnehmbar bzw. auswechselbar ausgebildet, so dass sich mehrere Personen ein Gerät teilen können und/oder defekte Aufsätze durch neue einfach und schnell ersetzt werden können. Ein entsprechendes Ultraschall-Zahnbehandlungsgerät mit einem auswechselbaren Zahnbürstenaufsatz ist beispielsweise aus der Druckschrift EP 2 637 600 B1 bekannt.

Ferner sind manuell oder elektrisch betätigbare Zungenreiniger für die Mundhygiene bekannt. Diese dienen zum Reinigen der Zunge von Bakterien, abgestorbenen Zellen und/oder Verschmutzung in Form von Speiseresten.

Beispielsweise finden bereits Zahnbürsten, insbesondere auch elektrische Zahnbürsten Verwendung, welche eine zusätzliche Zungenreinigungsfunktion aufweisen. Hierzu sind auf der Rückseite des Bürstenkopfes unterschiedlich geformte, von der Rückseite nach außen wegstehende Profilelemente vorgesehen, die zur Reinigung der Zunge vorgesehen sind.

Aus der CN 110 314 006 A eine elektrische Ultraschall-Zahnbürste bekannt, die eine in die Zahnbürste integrierte Blasanordnung zur Bereitstellung Luftstromes aufweist.

Ferner offenbart die DE 10 2019 000 770 A1 eine Hygienevorrichtung zur Verbesserung der Mundhygiene, dieein Mundstück mit einem Hohlraum zur Aufnahme einer Flüssigkeit aufweist, wobei die Flüssigkeit durch Poren und/oder Rillen an der Oberfläche des Mundstückes in den Mundraum abgegeben werden kann.

Der Druckschrift EP 3190991 B1 ist ein Ultraschall-Zungenreinigungsgerät zu entnehmen, welches ein Griffteil und ein darauf aufsteckbares Aufsteckteil zur Zungenreinigung mittels Ultraschall aufweist. Das Aufstecksteil weist dabei einen Kopfabschnitt mit darin seitlich aufgenommenen Ultraschallwandler auf, mittels dem Ultraschalldruckwellen erzeugt werden. Die erzeugten Ultraschalldruckwellen dienen zur "Reinigung" der Zungenoberfläche von bestehender Verschmutzung, insbesondere Bakterien oder Bakterienkolonien, welche häufig auch einen unangenehmen Mundgeruch verursachen. Nachteilig ist das bekannte Gerät nur zur Zungenbehandlung bzw. Zungenreinigung vorgesehen.

Ausgehend vom aufgezeigten Stand der Technik liegt der Erfindung die Aufgabe zugrunde, ein Ultraschall-Behandlungsgerät anzugeben, welches sowohl zur Zahn- als auch zur Zungenreinigung mittels Ultraschall verwendbar ist und welches eine effektive Reinigung der Zunge ermöglicht. Ebenfalls ist es Aufgabe der Erfindung einen Zungenbehandlungsaufsatz bereitzustellen, welcher zur Zungenreinigung ausgebildet ist und mit dem Handstück einer Ultraschall-Behandlungsgerätes zur Zahnreinigung kompatibel ist.

Die Aufgabe wird durch ein Ultraschall-Behandlungsgerät gemäß Patentanspruch 1 gelöst. Die Aufgabe wird auch durch einen Zungenbehandlungsaufsatz gemäß Patentanspruch 10 gelöst.

Der wesentliche Aspekt des erfindungsgemäß Ultraschall-Behandlungsgerätes ist darin zu sehen, der Behandlungsaufsatz als abnehmbarer Zungenbehandlungsaufsatz mit einem flachen Kopfabschnitt ausgebildet ist, dass der flache Kopfabschnitt eine die Behandlungsfläche bildenden Vorderseite und eine dieser gegenüberliegende Rückseite aufweist, und dass der Zungenbehandlungsaufsatz eine zur Rückseite des flachen Kopfabschnittes geöffnete Ausnehmung zur Aufnahme des Ultraschallwandlers aufweist, wobei zum hermetisch dichten Verschluss der Ausnehmung ein elastisches Verschlusselement vorgesehen ist und das elastische Verschlusselement einen elastischen Verschlussdeckel bildet, der aus einem gummiartigen Kunststoffmaterial hergestellt ist. Besonders vorteilhaft bildet das elastische Verschlusselement ein flexibles Massenelement auf, welche zum einen flüssigkeitsdichten Verschluss der Ausnehmung gewährleistet, zum anderen ein Schwingen des im stabilen bzw. steifen Gehäuse des Kopfabschnittes aufgenommenen Ultraschallwandlers ermöglicht.

Besonders vorteilhaft ist der elastische Verschlussdeckel einstückig ausgebildet. Vorteilhaft ist dieser elastische Verschlussdeckel schnell und werkzeuglos in die Ausnehmung einbringbar und auch aus dieser wieder entnehmbar, und zwar durch einfaches manuelles Eindrücken in die Ausnehmung.

Der flache Kopfabschnitt ist in einer bevorzugten Ausführungsvariante des Zungenbehandlungsaufsatzes zumindest abschnittsweise scheibenförmig ausgebildet, wobei vorzugsweise ein erster und zweiter, teilkreisscheibenförmiger Kopfabschnitt vorgesehen sind, die ineinander übergeben und unterschiedliche Durchmesser aufweisen. Dieser scheibenförmige Kopfabschnitt ermöglicht eine besonders effektive Reinigung der Zunge und ist auch benutzerfreundlich in den Mundraum einführbar.

Weiterhin vorteilhaft weist die Vorderseite des flachen Kopfabschnittes eine zum Entfernen von oberflächlich an der Zunge anhafteten Verschmutzung ausgebildete Profilierung auf. In einer bevorzugten Ausführungsvariante weist diese mehrere von Behandlungsfläche nach außen nasenartig abstehende Profilierungselemente auf, die vorzugsweise einstückig mit dem flachen Kopfabschnitt ausgebildet sein können. Auch können mehrere derartiger Profilierungselemente in Gruppen angeordnet sein und/oder in Draufsicht punktförmig, kreisförmig und/oder kreisringförmig ausgebildet sein. Durch das Vorsehen unterschiedlich geformter und flächig verteilter Profilierungselemente ist ein besonders zuverlässiges Entfernen von groben Verschmutzungen der Zunge möglich.

Der Ultraschallwandler ist bevorzugt ein elektroakustischer Piezo-Wandler, der zugeführte elektrische Schwingungsenergie in mechanische Schwingungsenergie, und zwar in Form Ultraschalldruckwellen umwandelt. Vorzugsweise ist der Ultraschallwandler zur Erzeugung von Ultraschalldruckwellen mit einer Frequenz von 350 kHz bis 450 kHz ausgebildet, wobei die Resonanzfrequenz des Ultraschallwandlers (4) vorzugsweise zwischen 405 kHz und 425 kHz beträgt. Die Piezo-Kapazität des Ultraschallwandlers beträgt dabei zwischen 5 nF und 8 nF, vorzugsweise zwischen 5,5 nF und 6 nF. Der Reinigungseffekt einer mittels diesen Ultraschallwandlers erzeugte Ultraschalldruckwelle ist besonders hoch.

In einer Weiterbildung der Erfindung ist die Handstückeinheit zum wahlweisen Betrieb des abnehmbaren Zungenbehandlungsaufsatzes als auch eines abnehmbaren Zahnbehandlungsaufsatzes eingerichtet. Aufgrund der Abstimmung der sowohl im Zahn- als auch im Zungenbehandlungsaufsatz verwendeten Ultraschallerzeuger bzw. Ultraschallwandler ist ohne technischen Mehraufwand die Handstückeinheit sowohl zur Zahn- als auch zur Zungenreinigung verwendbar, und zwar lediglich durch Wechseln des Aufsatzes.

Ebenfalls ist Gegenstand er Erfindung ein Zungenbehandlungsaufsatz zur Verwendung an einem Ultraschall-Behandlungsgerät wie zuvor beschrieben.

Weiterbildungen, Vorteile und Anwendungsmöglichkeiten der Erfindung ergeben sich auch aus der nachfolgenden Beschreibung von Ausführungsbeispielen und aus den Figuren. Dabei sind alle beschriebenen und/oder bildlich dargestellten Merkmale für sich oder in beliebiger Kombination grundsätzlich Gegenstand der Erfindung, unabhängig von ihrer Zusammenfassung in den Ansprüchen oder deren Rückbeziehung. Auch wird der Inhalt der Ansprüche zu einem Bestandteil der Beschreibung gemacht.

Die Erfindung wird im Folgenden anhand der Figuren an Ausführungsbeispielen näher erläutert. Es zeigen:
- Fig. 1: eine perspektivische Seitenansicht eines erfindungsgemäßen Ultraschall-Behandlungsgerätes mit einem Zungenbehandlungsaufsatz,

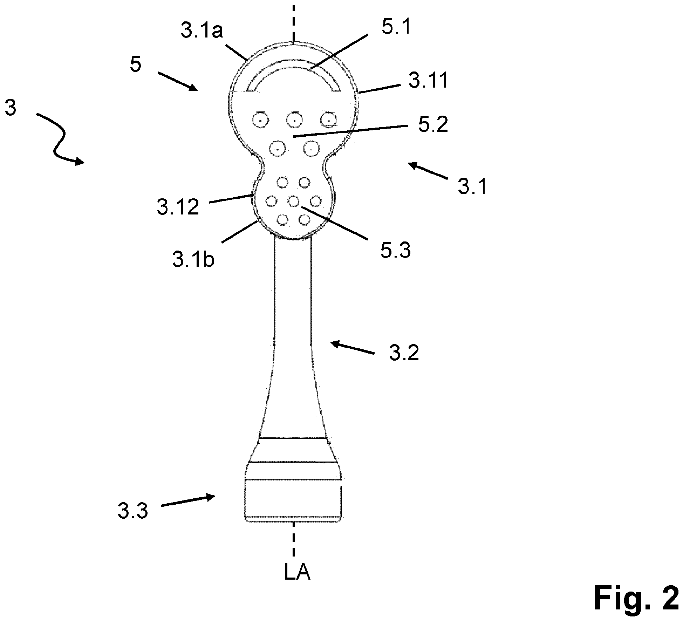

- Fig. 2: eine schematische Ansicht der Vorderseite eines erfindungsgemäßen Zungenbehandlungsaufsatzes, und
- Fig. 3: eine schematische Ansicht der Rückseite eines erfindungsgemäßen Zungenbehandlungsaufsatzes.

Für gleiche oder gleich wirkende Elemente der Erfindung werden in den Figuren identische Bezugszeichen verwendet. Ferner werden der Übersichtlichkeit halber nur Bezugszeichen in den einzelnen Figuren dargestellt, die für die Beschreibung der jeweiligen Figur erforderlich sind. Auch ist die Erfindung in den Figuren anhand schematischer Ansichten zur Erläuterung des grundlegenden Prinzips der Erfindung beispielhaft dargestellt.

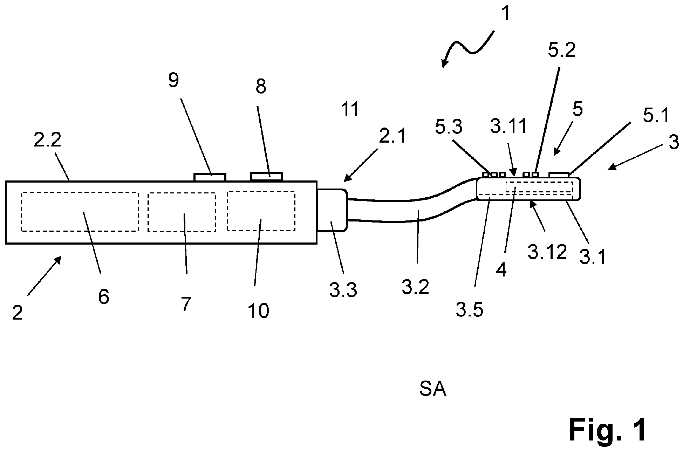

In Figur 1 ist in eine perspektivische Seitenansicht eines erfindungsgemäßen Ultraschall-Behandlungsgerätes 1 zur Zahn- und Zungenreinigung beispielhaft dargestellt.

Das erfindungsgemäße Ultraschall-Behandlungsgerät 1 umfasst eine Handstückeinheit 2 und zumindest einen abnehmbaren Behandlungsaufsatz 3, wobei der abnehmbare Behandlungsaufsatz 3 einen Kopfabschnitt 3.1, einen daran anschließend länglichen Verbindungsabschnitt 3.2 und einen Kupplungsabschnitt 3.3 aufweist.

Das Ultraschall-Behandlungsgerät 1 ist zum Betrieb eines abnehmbaren Behandlungsaufsatzes 3 zur Zungenreinigung in Form eines

Zungenbehandlungsaufsatzes und zum Betrieb zumindest eines weiteren Behandlungsaufsatzes zur Zahnreinigung in Form eines Zahnbehandlungsaufsatzes ausgebildet. Die Kupplungsabschnitte 3.3 des Zungenbehandlungsaufsatzes und des Zahnbehandlungsaufsatzes sind jeweils zur Herstellung einer elektromechanischen Verbindung mit der Handstückeinheit 2 ausgebildet, und zwar vorzugsweise in Form einer elektromechanischen Steckkupplungsverbindung oder Rastkupplungsverbindung. Der jeweilige Kupplungsabschnitt 3.3 steht hierzu in zumindest einer mechanischen Wirkverbindung mit dem freien Endabschnitt der Handstückeinheit 2, und zwar besteht im montierten Zustand eine kraft- und/oder formschlüssige Verbindung. Zugleich wird bei Herstellung dieser mechanischen Verbindung auch eine elektrische Verbindung zwischen der Handstückeinheit 2 und dem jeweiligen Behandlungsaufsatz 3 bzw. der im Behandlungsaufsatz 3 vorgesehenen elektrischen Komponenten hergestellt, und zwar vorzugsweise um diese mit dem für deren Betrieb erforderlichen elektrischen Energie zu versorgen. Üblicherweise sind hierzu zwei elektrische männliche Steckkontakte in der Handstückeinheit 2 oder im Behandlungsaufsatz 3 im Verbindungsbereich angeordnet sind und die zur Herstellung der elektrischen Verbindung mit entsprechenden weiblichen Steckkontakten verbindbar sind. Über im Behandlungsaufsatz 3 vom Kupplungsabschnitt 3.3 und den Verbindungsabschnitt 3.2 zum Kopfabschnitt 3 geführte elektrische Leitungen erfolgt dann die Übertragung der elektrischen Energie an den jeweiligen elektrischen Verbraucher, im vorliegenden Ausführungsbeispiel einem Ultraschallwandler 4, der sowohl in einem Zungenbehandlungsaufsatz als auch in einem Zahnbehandlungsaufsatz zum Einsatz kommt.

Im vorliegenden Ausführungsbeispiel wird jedoch ausschließlich auf die Ausgestaltung des Zungenbehandlungsaufsatzes näher angegangen, wobei auf eine detaillierte Beschreibung und Darstellung des elektromechanischen Verbindungsbereiches in den Figuren verzichtet wird.

Der Kupplungsabschnitt 3.3 des Zungenbehandlungsaufsatzes 3 ist im vorliegenden Ausführungsbeispiel zur Herstellung der elektromechanischen Verbindung an einen stirnseitigen Ankupplungsabschnitt 2.1 der Handstückeinheit 2 ankoppelbar, vorzugsweise darauf aufsteckbar. Ferner weist der jeweilige Behandlungsaufsatz 3 im Bereich des Kopfabschnittes 3.1 einen Ultraschallwandler 4 zur Erzeugung von Ultraschalldruckwellen auf. Der Behandlungsaufsatz 3 ist vorzugsweise einstückig ausgebildet und beispielsweise aus Kunststoff oder einen sonstigen geeigneten Material mit der erforderlichen Steifigkeit bei geringen Gewicht hergestellt.

Zur Versorgung des Ultraschallwandlers 4 mit elektrischer Energie sowie zur Ansteuerung dessen sind in der Handstückeinheit 2 zumindest eine Energieversorgungseinheit 6 und eine Steuereinheit 7 vorgesehen. Hierzu weist die Handstückeinheit 2 ein vorzugsweise zylinderförmiges Gehäuse 2.1, vorzugsweise Kunststoffgehäuse mit einem hermetisch dicht verschlossenen Innenraum auf, in dem die Energieversorgungseinheit 6 und die Steuereinheit 7 aufgenommen sind. In dem Gehäuse 2.1 der Handstückeinheit 2 können auch noch weitere, in den Figuren nicht dargestellte Funktionselemente des Ultraschall-Behandlungsgerätes 1 untergebracht sein.

Auch kann die Handstückeinheit 2 ein mit der Steuereinheit 7 verbundenes Bedienelement 8 aufweisen, über welches das Ultraschall-Behandlungsgerät 1 ein- und ausgeschaltet werden kann. Auch können noch weitere Betätigungselemente 9 vorgesehen sein, die zur Einstellung ggf. vom Gerät 1 bereitgestellter Betriebsmodi und/oder der Intensität der Behandlung vorgesehen sind.

Des Weiteren kann die Handstückeinheit 2 auch eine in den Figuren nicht näher gezeigte Anzeigeeinheit verfügen, über welche einem Benutzer beispielsweise der aktuelle Betriebszustand, Betriebsmodus und/oder die Intensität der Behandlung angezeigt werden. Es versteht sich, dass das Ultraschall-Behandlungsgerät 1 auch ein oder mehrere Schnittstellen aufweisen kann, die drahtlos oder drahtgebunden ausgebildet sein können. Auch kann im Gehäuse eine Speichereinheit 10 aufgenommen sein, die mit der Steuereinheit 7 verbunden ist und in Daten und/oder Steuerroutinen speicherbar sind.

Erfindungsgemäß weist das Ultraschall-Behandlungsgerät 1 einen abnehmbaren Zungenbehandlungsaufsatz 3 mit einem flachen Kopfabschnitt 3.1 auf, wobei der flache Kopfabschnitt 3.1 eine die Behandlungsfläche des Aufsatzes 3 bildenden Vorderseite 3.11 und eine dieser gegenüberliegende Rückseite 3.12 umfasst. Die Vorderseite 3.11 des flachen Kopfabschnittes 3.1 weist eine zum Entfernen von oberflächlich an der Zunge anhafteten Verschmutzungen ausgebildete Profilierung 5 auf. Schließlich weist der Zungenbehandlungsaufsatz 3 eine zur Rückseite 3.12 des flachen Kopfabschnittes 3.1 geöffnete Ausnehmung 3.4 zur Aufnahme des Ultraschallwandlers 4 auf, wobei zum hermetisch dichten Verschluss der Ausnehmung 3.4 ein elastisches Verschlusselement 3.5 vorgesehen ist. Weiterhin ist das elastische Verschlusselement 3.5 in Form eines elastischen Verschlussdeckels gebildet, welcher in die Ausnehmung 3.4 eingreift, und zwar vorzugsweise formschlüssig in diese eingesetzt wird. Dadurch entsteht ein hermetisch dichter Verschluss der Ausnehmung 3.4 und ein Schutz vor Feuchtigkeit des in der Ausnehmung 3.4 aufgenommenen Ultraschallwandlers 4 sowie von dessen elektrischen Anschlussbauteilen.

Der Ultraschallwandler 4 ist vorzugsweise flach und scheibenförmig ausgebildet und in Form eines Piezo-Ultraschallwandlers realisiert. Dieser verfügt beispielsweise über zwei elektrische Anschlussleitungen, die durch den Verbindungsabschnitt 3.2 zum Kupplungsabschnitt 3.3 geführt sind. Der Ultraschallwandler 4 ist ein elektroakustischer Piezo-Wandler, welcher die zugeführte elektrische Energie bzw. Schwingungsenergie in in mechanische Energie bzw. Schwingungsenergie, und zwar in Form von Schallwellen, insbesondere Ultraschallwellen bzw. Ultraschalldruckwellen umwandelt. Hierzu weist dieser zumindest ein aktives Piezoelement auf, welches in einem Gehäuse aufgenommen und mit den elektrischen Anschlussleitungen verbunden ist. Der Ultraschallwandler 4 ist vorzugsweise zur Erzeugung von Ultraschalldruckwellen mit einer Frequenz von 350 kHz bis 450 kHz bei einer elektrischen Betriebsspannung zwischen 18 und 20 Volt ausgebildet. Die Resonanzfrequenz des Ultraschallwandlers 4 beträgt vorzugsweise zwischen 405 kHz und 425 kHz. Die Piezo-Kapazität des Ultraschallwandlers 4 liegt zwischen 5 nF und 8 nF, vorzugsweise zwischen 5,5 nF und 6 nF. Die elektrischen Betriebsparameter des Ultraschallwandlers 4 entsprechen vorzugsweise denjenigen, die auch in Zahnbehandlungsaufsätzen Verwendung finden, so dass keine Anpassung der Energieversorgungseinheit 6 und/oder Steuereinheit 7 der Handstückeinheit 2 beim einem Betriebswechsel vom Zahnbehandlungsaufsatz zum Zungenbehandlungsaufsatz erforderlich ist.

Das Verschlusselement 3.5 ist einstückig ausgebildet und aus einem elastischen Kunststoffmaterial, erfindungsgemäß einem gummiartigen Kunststoffmaterial hergestellt. Die Querschnittsform des elastischen Verschlusselementes 3.5 entspricht im Wesentlichen der Querschnittsform der Ausnehmung 3.4 in der Rückseite 3.12 des Kopfabschnittes 3.1. Die Materialstärke des Verschlusselement 3.5 ist derart gewählt, dass der nach Aufnahme des Ultraschallwandlers 4 in der Ausnehmung 3.4 verbleibende Innenraum möglichst vollständig ausgefüllt ist, d.h. der Ultraschallwandler 4 zwischen der die Vorderseite 3.11 bildenden Wandung des Gehäuses 2.1 und dem in die Ausnehmung 3.4 eingebrachten Verschlusselementes 3.5 aufgenommen ist, vorzugsweise klemmend gehalten ist. Die elastische Ausgestaltung der Verschlusselementes 3.5 unterstützt ein Schwingen des Ultraschallwandlers 4, zumal die die Vorderseite 3.11 bildenden Wandung des Gehäuses 2.1 im Wesentlichen steif ist. Das Verschlusselement 4 bildet ein flexibles Massenelement aus, welche der stabilen bzw. steifen Wandung gegenüberliegt und somit ein Schwingen des vorzugsweise daran anliegenden Ultraschallwandlers 4 freigibt.

Die auf der Vorderseite 3.11 des Zungenbehandlungsaufsatzes 3 vorgesehen Profilierung 5 kann beispielsweise mehrere von der Behandlungsfläche bzw. Vorderseite 3.11 nach außen nasenartig abstehende Profilierungselemente 5.1, 5.2, 5.3 oder Gruppen von Profilierungselementen 5.1, 5.2, 5.3 aufweisen, die vorzugsweise jeweils einstückig mit dem flachen Kopfabschnitt 3.1 ausgebildet sind.

Im vorliegenden Ausführungsbeispiel sind erste bis dritte Profilierungselemente 5.1, 5.2, 5.3 vorgesehen, wobei das erste Profilierungselement 5.1 in Draufsicht kreisbogenförmig ausgebildet ist, d.h. das erste Profilierungselement 5.1 bildet einen von der Behandlungsfläche nach außen nasenartig abstehenden, kreisbogenförmigen Vorsprung aus. Die zweiten und dritten Profilierungselemente 5.2, 5.3 sind punkt- bzw. kreisförmig ausgebildet, wobei mehrere zweite Profilierungselemente 5.2 entlang zweier parallel zueinander und senkrecht zur Längsachse LA des Zungenbehandlungsaufsatzes 3 angeordnet sind und in Form von Kugelsegmenten realisiert sind.

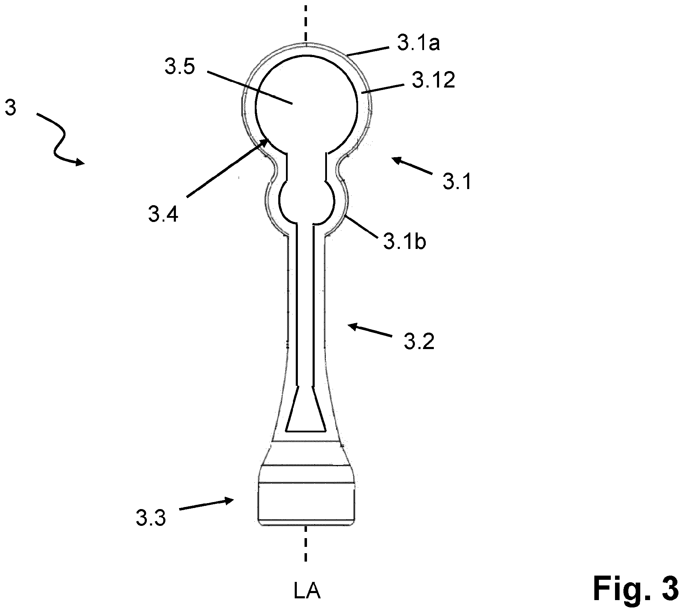

Beispielhaft ist in Figur 3 eine Gruppe von fünf zweiten Profilierungselementen 5.2 gezeigt, die sich an das erste, kreisbogenförmige Profilierungselement 5.1 entlang der Längsachse LA anschließt, und zwar gefolgt von dritten Profilierungselementen 5.3, die ebenfalls in Draufsicht kreisförmig bzw. kreissegmentartige ausgebildet sind. Auch hier ist eine Gruppe von sieben dritten Profilierungselemente 5.3 vorgesehen, wobei eines der dritten Profilierungselemente 5.3 einen Mittelpunkt bildet und die weiteren dritten Profilierungselemente 5.3 näherungsweise gleichmäßig um diesen Mittelpunkt und beabstandet davon auf einer fiktiven Kreisbahn angeordnet sind.

Sämtliche der genannten Profilierungselemente 5.1, 5.2, 5.3 sind spiegelsymmetrisch zu einer die Längsachse LA aufnehmenden Ebene ausgebildet. Die kreisförmigen bzw. kreissegmentartigen zweiten und dritten Profilierungselemente 5.2, 5.3 weisen vorzugsweise einen unterschiedlichen Durchmesser auf.

Besonders bevorzugt ist der flache Kopfabschnitt 3.1 des Zungenbehandlungsaufsatzes 3 zumindest abschnittsweise scheibenförmig ausgebildet ist, um eine möglichst benutzerfreundliche Anwendung dessen im Mundraum zu gewährleisten.

In der gezeigten Ausführungsvariante weist der flache Kopfabschnitt 3.1 einen ersten und zweiten, teilkreisscheibenförmigen Kopfabschnitt 3.1a, 3.1b auf, die ineinander übergeben und unterschiedliche Durchmesser aufweisen. Der Durchmesser des ersten Kopfabschnittes 3.1a entspricht näherungsweise dem doppelten Durchmesser des zweiten Kopfabschnittes 3.1b, der in den Verbindungsabschnitt 3.2 übergeht. Besonders bevorzugt sind die ersten und zweiten Profilierungselemente 5.1, 5.2 im Bereich des ersten Kopfabschnittes 3.1a und die dritten Profilierungselemente 5.3 im Bereich des zweiten Kopfabschnittes 3.1b angeordnete, wodurch eine abgestufte Reinigung der Zunge von grob nach fein möglich ist.

Auch kann sich die Ausnehmung 3.4 über den Kopfabschnitt 3.1 in den Verbindungsabschnitt 3.2 erstrecken, die vollständig mit dem elastischen Verschlussdeckel 3.5 verschließbar ist. Bei der in Figur 3 gezeigten Ausführungsvariante erstreckt sich die Ausnehmung 3.4 beispielsweise über den ersten und zweiten Kopfabschnitt 3.1a, 3.1b und den Verbindungsabschnitt 3.2 entlang der Längsachse LA, und zwar näherungsweise bis kurz vor dem Kupplungsabschnitt 3.3.

Die Erfindung wurde voranstehend an Ausführungsbeispielen beschrieben. Es versteht sich, dass zahlreiche Änderungen sowie Abwandlungen möglich sind, ohne dass dadurch der der Erfindung zugrundeliegend Erfindungsgedanke verlassen wird.

### Bezugszeichenliste

- 1: Ultraschall-Behandlungsgerät
- 2: Handstückeinheit
- 3: Behandlungsaufsatz bzw. Zungenbehandlungsaufsatz
- 3.1: Kopfabschnitt
- 3.1a: erster Kopfabschnitt
- 3.1b: zweiter Kopfabschnitt
- 3.11: Vorderseite
- 3.12: Rückseite
- 3.2: Verbindungsabschnitt
- 3.3: Kupplungsabschnitt
- 3.4: Ausnehmung
- 3.5: Verschlusselement
- 4: Ultraschallwandler
- 5: Profilierung
- 5.1: erste Profilierungselemente
- 5.2: zweite Profilierungselemente
- 5.3: dritte Profilierungselemente
- 6: Energieversorgungseinheit
- 7: Steuereinheit
- 8: Bedienelement
- 9: weiteres Bedienelement
- 10: Speichereinheit

- LA: Längsachse

## Patentansprüche

1. Ultraschall-Behandlungsgerät (1) umfassend eine Handstückeinheit (2) und zumindest einen abnehmbaren Behandlungsaufsatz (3), der einen Kopfabschnitt (3.1), einen daran anschließend länglichen Verbindungsabschnitt (3.2) und einen Kupplungsabschnitt (3.3) aufweist, wobei der Kupplungsabschnitt (3.3) zur Herstellung einer elektromechanischen Verbindung mit der Handstückeinheit (2) ausgebildet ist und der Behandlungsaufsatz (3) im Bereich des Kopfabschnittes (3.1) einen Ultraschallwandler (4) zur Erzeugung von Ultraschalldruckwellen aufweist, bei dem der Behandlungsaufsatz (3) als abnehmbarer Zungenbehandlungsaufsatz mit einem flachen Kopfabschnitt (3.1) ausgebildet ist, bei dem der flache Kopfabschnitt (3.1) eine die Behandlungsfläche bildende Vorderseite (3.11) und eine dieser gegenüberliegende Rückseite (3.12) aufweist, und bei dem der Zungenbehandlungsaufsatz (3) eine zur Rückseite (3.12) des flachen Kopfabschnittes (3.1) geöffnete Ausnehmung (3.4) zur Aufnahme des Ultraschallwandlers (4) aufweist, wobei zum hermetisch dichten Verschluss der Ausnehmung (3.4) ein elastisches Verschlusselement (3.5) vorgesehen ist und das elastische Verschlusselement (3.5) einen elastischen Verschlussdeckel bildet, **dadurch gekennzeichnet dass** der Verschlussdeckel aus einem gummiartigen Kunststoffmaterial hergestellt ist.

2. Ultraschall-Behandlungsgerät (1) nach Anspruch 1, **dadurch gekennzeichnet, dass** der elastische Verschlussdeckel (3.5) einstückig ausgebildet ist.

3. Ultraschall-Behandlungsgerät (1) nach Anspruch 1 oder 2, **dadurch gekennzeichnet, dass** der flache Kopfabschnitt (3.1) des Zungenbehandlungsaufsatzes (3) zumindest abschnittsweise scheibenförmig ausgebildet ist.

4. Ultraschall-Behandlungsgerät (1) nach Anspruch 3, **dadurch gekennzeichnet, dass** der flache Kopfabschnitt (3.1) einen ersten und zweiten, teilkreisscheibenförmigen Kopfabschnitt (3.1a, 3.1b) aufweist, die ineinander übergehen und unterschiedliche Durchmesser aufweisen.

5. Ultraschall-Behandlungsgerät (1) nach einem der vorhergehenden Ansprüche, **dadurch gekennzeichnet, dass** die Vorderseite (3.11) des flachen Kopfabschnittes (3.1) eine zum Entfernen von oberflächlich an der Zunge anhafteten Verschmutzung ausgebildete Profilierung (5) aufweist.

6. Ultraschall-Behandlungsgerät (1) nach Anspruch 5, **dadurch gekennzeichnet, dass** die Profilierung (5) mehrere von Behandlungsfläche nach außen nasenartig abstehende Profilierungselemente (5.1, 5.2, 5.3) aufweist, die einstückig mit dem flachen Kopfabschnitt (3.1) ausgebildet sind, wobei die Profilierungselemente (5.1, 5.2, 5.3) in Draufsicht punktförmig, kreisförmig und/oder kreisringförmig ausgebildet sind.

7. Ultraschall-Behandlungsgerät (1) nach einem der vorhergehenden Ansprüche, **dadurch gekennzeichnet, dass** der Ultraschallwandler (4) ein elektroakustischer Piezo-Wandler ist, der zugeführte elektrische Schwingungsenergie in mechanische Schwingungsenergie, und zwar in Form von Ultraschalldruckwellen umwandelt oder dass der Ultraschallwandler (4) zur Erzeugung von Ultraschalldruckwellen mit einer Frequenz von 350 kHz bis 450 kHz ausgebildet ist, wobei die Resonanzfrequenz des Ultraschallwandlers (4) vorzugsweise zwischen 405 kHz und 425 kHz beträgt.

8. Ultraschall-Behandlungsgerät (1) nach Anspruch 7, **dadurch gekennzeichnet, dass** die Piezo-Kapazität des Ultraschallwandlers (4) zwischen 5 nF und 8 nF, vorzugsweise zwischen 5,5 nF und 6 nF, beträgt.

9. Ultraschall-Behandlungsgerät (1) nach einem der vorhergehenden Ansprüche, **dadurch gekennzeichnet, dass** die Handstückeinheit (2) zum wahlweisen Betrieb des abnehmbaren Zungenbehandlungsaufsatzes (3) als auch eines abnehmbaren Zahnbehandlungsaufsatzes eingerichtet ist.

10. Zungenbehandlungsaufsatz (3) zur Verwendung an einem Ultraschall-Behandlungsgerät (1) nach einem der vorhergehenden Ansprüche mit einem Kopfabschnitt (3.1), einem daran anschließend länglichen Verbindungsabschnitt (3.2) und einem Kupplungsabschnitt (3.3), wobei der Kupplungsabschnitt (3.3) zur Herstellung einer elektromechanischen Verbindung mit der Handstückeinheit (2) ausgebildet ist und der Zungenbehandlungsaufsatz (3) im Bereich des Kopfabschnittes (3.1) einen Ultraschallwandler (4) zur Erzeugung von Ultraschalldruckwellen aufweist, bei dem der flache Kopfabschnitt (3.1) eine die Behandlungsfläche bildende Vorderseite (3.11) und eine dieser gegenüberliegende Rückseite (3.12) aufweist, und bei dem der Zungenbehandlungsaufsatz (3) eine zur Rückseite (3.12) des flachen Kopfabschnittes (3.1) geöffnete Ausnehmung (3.4) zur Aufnahme des Ultraschallwandlers (4) aufweist, wobei zum hermetisch dichten Verschluss der Ausnehmung (3.4) ein elastisches Verschlusselement (3.5) vorgesehen ist und das elastische Verschlusselement (3.5) einen elastischen Verschlussdeckel bildet, **dadurch gekennzeichnet dass** der Verschlussdeckel aus einem gummiartigen Kunststoffmaterial hergestellt ist.

11. Zungenbehandlungsaufsatz (3) nach Anspruch 10, **dadurch gekennzeichnet, dass** der elastische Verschlussdeckel (3.5) einstückig ausgebildet ist.

12. Zungenbehandlungsaufsatz (3) nach Anspruch 10 oder 11, **dadurch gekennzeichnet, dass** der flache Kopfabschnitt (3.1) des Zungenbehandlungsaufsatzes (3) zumindest abschnittsweise scheibenförmig ausgebildet ist und/oder dass der flache Kopfabschnitt (3.1) einen ersten und zweiten, teilkreisscheibenförmigen Kopfabschnitt (3.1a, 3.1b) aufweist, die ineinander übergehen und unterschiedliche Durchmesser aufweisen.

13. Zungenbehandlungsaufsatz (3) nach einem der Ansprüche 10 bis 12, **dadurch gekennzeichnet, dass** die Vorderseite (3.11) des flachen Kopfabschnittes (3.1) eine zum Entfernen von oberflächlich an der Zunge anhafteten Verschmutzung ausgebildete Profilierung (5) aufweist.

14. Zungenbehandlungsaufsatz (3) nach Anspruch 13, **dadurch gekennzeichnet, dass** die Profilierung (5) mehrere von Behandlungsfläche nach außen nasenartig abstehende Profilierungselemente (5.1, 5.2, 5.3) aufweist, die einstückig mit dem flachen Kopfabschnitt (3.1) ausgebildet sind, wobei die Profilierungselemente (5.1, 5.2, 5.3) in Draufsicht punktförmig, kreisförmig und/oder kreisringförmig ausgebildet sind.

15. Zungenbehandlungsaufsatz (3) nach einem der Ansprüche 10 bis 14, **dadurch gekennzeichnet, dass** der Ultraschallwandler (4) ein elektroakustischer Piezo-Wandler ist, der zugeführte elektrische Schwingungsenergie in mechanische Schwingungsenergie, und zwar in Form von Ultraschalldruckwellen umwandelt.

16. Zungenbehandlungsaufsatz (3) nach einem der Ansprüche 10 bis 15, **dadurch gekennzeichnet, dass** der Ultraschallwandler (4) zur Erzeugung von Ultraschalldruckwellen mit einer Frequenz von 350 kHz bis 450 kHz ausgebildet ist, wobei die Resonanzfrequenz des Ultraschallwandlers (4) vorzugsweise zwischen 405 kHz und 425 kHz beträgt.

17. Zungenbehandlungsaufsatz (3) nach einem der Ansprüche 10 bis 16, **dadurch gekennzeichnet, dass** die Piezo-Kapazität des Ultraschallwandlers (4) zwischen 5 nF und 8 nF, vorzugsweise zwischen 5,5 nF und 6 nF, beträgt.

## Claims

1. Ultrasonic treatment apparatus (1) comprising a handpiece unit (2) and at least one removable treatment attachment (3), which comprises a head section (3.1), an adjoining elongate connecting section (3.2) and a coupling section (3.3), wherein the coupling section (3.3) is configured for establishing an electromechanical connection to the handpiece unit (2) and the treatment attachment (3) comprises an ultrasonic transducer (4) in the region of the head section (3.1) for generating ultrasonic shockwaves, wherein the treatment attachment (3) is formed as a removable tongue treatment attachment with a flat head section (3.1), wherein the flat head section (3.1) comprises a front side (3.11) forming the treatment surface and a rear side (3.12) lying opposite said front side, and wherein the tongue treatment attachment (3) comprises a recess (3.4), which is open towards the rear side (3.12) of the flat head section (3.1), for receiving the ultrasonic transducer (4), wherein an elastic closing element (3.5) is provided for the hermetically tight closure of the recess (3.4) and the elastic closing element (3.5) forms an elastic closing cover, **characterized in that** the closing cover is made of a rubber-like plastic material.

2. Ultrasonic treatment apparatus (1) according to claim 1, **characterized in that** the elastic closing cover (3.5) is formed in one piece.

3. Ultrasonic treatment apparatus (1) according to claim 1 or 2, **characterized in that** the flat head section (3.1) of the tongue treatment attachment (3) is formed in a disk-shaped manner at least in sections.

4. Ultrasonic treatment apparatus (1) according to claim 3, **characterized in that** the flat head section (3.1) comprises a first and second, partially circular disk-shaped head section (3.1a, 3.1b), which merge into one another and which have different diameters.

5. Ultrasonic treatment apparatus (1) according to one of the preceding claims, **characterized in that** the front side (3.11) of the flat head section (3.1) comprises a profiling (5), which is designed for removing contaminations adhering to the tongue on the surface.

6. Ultrasonic treatment apparatus (1) according to claim 5, **characterized in that** the profiling (5) comprises several profiling elements (5.1, 5.2, 5.3), which protrude outwards in a nose-like manner from the treatment surface and which are formed integrally with the flat head section (3.1), wherein the profiling elements (5.1, 5.2, 5.3) are formed in a punctiform, circular and/or circular ring-shaped manner in the top view.

7. Ultrasonic treatment apparatus (1) according to one of the preceding claims, **characterized in that** the ultrasonic transducer (4) is an electroacoustic piezo transducer, which converts supplied electrical vibrational energy into mechanical vibrational energy, namely in the form of ultrasonic shockwaves or **in that** the ultrasonic transducer (4) is formed for generating ultrasonic shockwaves with a frequency of 350 kHz to 450 kHz, wherein the resonance frequency of the ultrasonic transducer (4) is preferably between 405 kHz and 425 kHz.

8. Ultrasonic treatment apparatus (1) according to claim 7, **characterized in that** the piezo capacitance of the ultrasonic transducer (4) is between 5 nF and 8 nF, preferably between 5.5 nF and 6 nF.

9. Ultrasonic treatment apparatus (1) according to one of the preceding claims, **characterized in that** the handpiece unit (2) is configured for selectively operating the removable tongue treatment attachment (3) as well as of a removable tooth treatment attachment.

10. Tongue treatment attachment (3) for use on an ultrasonic treatment apparatus (1) according to one of the preceding claims with a head section (3.1), an adjoining elongate connecting section (3.2) and a coupling section (3.3), wherein the coupling section (3.3) is formed for establishing an electromechanical connection to the handpiece unit (2) and the tongue treatment attachment (3) comprises an ultrasonic transducer (4) in the region of the head section (3.1) for generating ultrasonic shockwaves, wherein the flat head section (3.1) comprises a front side (3.11) forming the treatment surface and a rear side (3.12) lying opposite said front side, and wherein the tongue treatment attachment (3) comprises a recess (3.4), which is open towards the rear side (3.12) of the flat head section (3.1), for receiving the ultrasonic transducer (4), wherein an elastic closing element (3.5) is provided for the hermetically tight closure of the recess (3.4) and the elastic closing element (3.5) forms an elastic closing cover, **characterized in that** the closing cover is made of a rubber-like plastic material.

11. Tongue treatment attachment (3) according to claim 10, **characterized in that** the elastic closing cover (3.5) is formed in one piece.

12. Tongue treatment attachment (3) according to claim 10 or 11, **characterized in that** the flat head section (3.1) of the tongue treatment attachment (3) is formed in a disk-shaped manner at least in sections and/or that the flat head section (3.1) comprises a first and second, partially circular disk-shaped head section (3.1a, 3.1b), which merge into one another and which have different diameters.

13. Tongue treatment attachment (3) according to one of claims 10 to 12, **characterized in that** the front side (3.11) of the flat head section (3.1) comprises a profiling (5), which is designed for removing contaminations adhering to the tongue on the surface.

14. Tongue treatment attachment (3) according to claim 13, **characterized in that** the profiling (5) comprises several profiling elements (5.1, 5.2, 5.3), which protrude outwards in a nose-like manner from the treatment surface and which are formed integrally with the flat head section (3.1), wherein the profiling elements (5.1, 5.2, 5.3) are formed in a punctiform, circular and/or circular ring-shaped manner in the top view.

15. Tongue treatment attachment (3) according to one of claims 10 to 14, **characterized in that** the ultrasonic transducer (4) is an electroacoustic piezo transducer, which converts supplied electrical vibrational energy into mechanical vibrational energy, namely in the form of ultrasonic shockwaves.

16. Tongue treatment attachment (3) according to one of claims 10 to 15, **characterized in that** the ultrasonic transducer (4) is formed for generating ultrasonic shockwaves with a frequency of 350 kHz to 450 kHz, wherein the resonance frequency of the ultrasonic transducer (4) is preferably between 405 kHz and 425 kHz.

17. Tongue treatment attachment (3) according to one of claims 10 to 16, **characterized in that** the piezo capacitance of the ultrasonic transducer (4) is between 5 nF and 8 nF, preferably between 5.5 nF and 6 nF.

## Revendications

1. Appareil de traitement par ultrasons (1) comprenant une unité de pièce à main (2) et au moins un embout de traitement (3) amovible qui présente un tronçon de tête (3.1) et un tronçon de liaison longitudinal (3.2) se raccordant dessus et un tronçon de couplage (3.3), dans lequel le tronçon de couplage (3.3) est conçu pour créer une liaison électromécanique avec l'unité de pièce à main (2) et l'embout de traitement (3) présente au niveau du tronçon de tête (3.1) un transducteur d'ultrasons (4) pour produire des ondes de pression ultrasoniques, dans lequel l'embout de traitement (3) est conçu en tant qu'embout de traitement de la langue amovible avec un tronçon de tête plat (3.1), dans lequel le tronçon de tête plat (3.1) présente une face avant (3.11) formant la face de traitement et un dos (3.12) face à celui-ci et dans lequel l'embout de traitement de la langue (3) présente un évidement (3.4) ouvert en direction du dos (3.12) du tronçon de tête plat (3.1) pour recevoir le transducteur d'ultrasons (4), dans lequel un élément de fermeture élastique (3.5) est prévu pour la fermeture étanche et hermétique de l'évidement (3.4) et l'élément de fermeture élastique (3.5) forme un couvercle de fermeture élastique, **caractérisé en ce que** le couvercle de fermeture est fabriqué dans un matériau plastique caoutchouteux.

2. Appareil de traitement par ultrasons (1) selon la revendication 1, **caractérisé en ce que** le couvercle de fermeture élastique (3.5) est formé d'un seul bloc.

3. Appareil de traitement par ultrasons (1) selon la revendication 1 ou 2, **caractérisé en ce que** le tronçon de tête plat (3.1) de l'embout de traitement de la langue (3) est en forme de disque au moins par tronçons.

4. Appareil de traitement par ultrasons (1) selon la revendication 3, **caractérisé en ce que** le tronçon de tête plat (3.1) présente un premier et un second tronçon de tête (3.1a, 3.1b) en forme de disque circulaire partiel qui passent l'un dans l'autre et présentent des diamètres différents.

5. Appareil de traitement par ultrasons (1) selon l'une des revendications précédentes, **caractérisé en ce que** la face avant (3.11) du tronçon de tête plat (3.1) présente un profilé (5) formé pour enlever des salissures collées à la surface de la langue.

6. Appareil de traitement par ultrasons (1) selon la revendication 5, **caractérisé en ce que** le profilé (5) présente plusieurs éléments de profilé (5.1, 5.2, 5.3) faisant saillie de la face de traitement vers l'extérieur en forme d'ergot, qui sont formés d'un seul bloc avec le tronçon de tête plat (3.1), dans lequel les éléments de profilé (5.1, 5.2, 5.3) sont en forme de point, de cercle et/ou d'anneau de cercle en vue de dessus.

7. Appareil de traitement par ultrasons (1) selon l'une des revendications précédentes, **caractérisé en ce que** le transducteur d'ultrasons (4) est un convertisseur piezo électroacoustique qui convertit l'énergie vibratoire électrique amenée en énergie vibratoire mécanique et ce sous forme d'ondes de pression ultrasoniques ou que le transducteur d'ultrasons (4) est conçu pour produire des ondes de pression ultrasoniques avec une fréquence de 350 kHz à 450 kHz, dans lequel la fréquence de résonance du transducteur d'ultrasons (4) fait de préférence entre 405 kHz et 425 kHz.

8. Appareil de traitement par ultrasons (1) selon la revendication 7, **caractérisé en ce que** la capacité piezo du transducteur d'ultrasons (4) fait entre 5 nF et 8 nF, de préférence entre 5,5 nF et 6 nF.

9. Appareil de traitement par ultrasons (1) selon l'une des revendications précédentes, **caractérisé en ce que** l'unité de pièce à main (2) est conçue pour le fonctionnement au choix de l'embout de traitement de la langue (3) amovible en tant qu'un embout de traitement dentaire amovible également.

10. Embout de traitement de la langue (3) pour utilisation sur un appareil de traitement par ultrasons (1) selon l'une des revendications précédentes avec un tronçon de tête (3.1), un tronçon de liaison longitudinal (3.2) se raccordant dessus et un tronçon de couplage (3.3), dans lequel le tronçon de couplage (3.3) est conçu pour créer une liaison électromécanique avec l'unité de pièce à main (2) et l'embout de traitement (3) présente au niveau du tronçon de tête (3.1) un transducteur d'ultrasons (4) pour produire des ondes de pression ultrasoniques, dans lequel le tronçon de tête plat (3.1) présente une face avant (3.11) formant la face de traitement et un dos (3.12) face à celui-ci et dans lequel l'embout de traitement de la langue (3) présente un évidement (3.4) ouvert en direction du dos (3.12) du tronçon de tête plat (3.1) pour recevoir le transducteur d'ultrasons (4), dans lequel un élément de fermeture élastique (3.5) est prévu pour la fermeture étanche et hermétique de l'évidement (3.4) et l'élément de fermeture élastique (3.5) forme un couvercle de fermeture élastique, **caractérisé en ce que** le couvercle de fermeture est fabriqué dans un matériau plastique caoutchouteux.

11. Embout de traitement de la langue (3) selon la revendication 10, **caractérisé en ce que** le couvercle de fermeture élastique (3.5) est formé d'un seul bloc.

12. Embout de traitement de la langue (3) selon la revendication 10 ou 11, **caractérisé en ce que** le tronçon de tête plat (3.1) de l'embout de traitement de la langue (3) est en forme de disque au moins par tronçons et/ou que le tronçon de tête plat (3.1) présente un premier et un second tronçon de tête (3.1a, 3.1b) en forme de disque circulaire partiel qui passent l'un dans l'autre et présentent des diamètres différents.

13. Embout de traitement de la langue (3) selon l'une des revendications 10 à 12, **caractérisé en ce que** la face avant (3.11) du tronçon de tête plat (3.1) présente un profilé (5) formé pour enlever des salissures collées à la surface de la langue.

14. Embout de traitement de la langue (3) selon la revendication 13, **caractérisé en ce que** le profilé (5) présente plusieurs éléments de profilé (5.1, 5.2, 5.3) faisant saillie de la surface de traitement vers l'extérieur en forme d'ergot, qui sont formés d'un seul bloc avec le tronçon de tête plat (3.1), dans lequel les éléments de profilé (5.1, 5.2, 5.3) sont en forme de point, de cercle et/ou d'anneau de cercle en vue de dessus.

15. Embout de traitement de la langue (3) selon l'une des revendications 10 à 14, **caractérisé en ce que** le transducteur d'ultrasons (4) est un convertisseur piezo électroacoustique qui convertit l'énergie vibratoire électrique amenée en énergie vibratoire mécanique et ce sous forme d'ondes de pression ultrasoniques.

16. Embout de traitement de la langue (3) selon l'une des revendications 10 à 15, **caractérisé en ce que** le transducteur d'ultrasons (4) est conçu pour produire des ondes de pression ultrasoniques avec une fréquence de 350 kHz à 450 kHz, dans lequel la fréquence de résonance du transducteur d'ultrasons (4) fait de préférence entre 405 kHz et 425 kHz.

17. Embout de traitement de la langue (3) selon l'une des revendications 10 à 16, **caractérisé en ce que** la capacité piezo du transducteur d'ultrasons (4) fait entre 5 nF et 8 nF, de préférence entre 5,5 nF et 6 nF.
